# EUROPEAN PATENT SPECIFICATION

(11) **EP 2 872 108 B1**
(45) Date of publication and mention of the grant of the patent: **08.08.2018**
(21) Application number: 13728416.2
(22) Date of filing: 14.06.2013
(51) Int. Cl.: A61K 8/26, A61K 8/29, A61Q 11/00, A61K 8/02

(54) **TOOTH WHITENING ORAL CARE COMPOSITION**
ZÄHNEWEISSENDE MUNDPFLEGEZUSAMMENSETZUNG
COMPOSITION POUR L'HYGIÈNE BUCCALE ET LE BLANCHIMENT DES DENTS

(30) Priority: 10.07.2012 WO PCT/CN2012/000946; 17.08.2012 EP 12180813
(43) Date of publication of application: 20.05.2015
(73) Proprietor: Unilever N.V., 3013 AL Rotterdam (NL); Unilever PLC, London, Greater London EC4Y 0DY (GB)
(72) Inventor: DENG, Yan, Shanghai 200335 (CN); DING, Guanjun, Shanghai 200050 (CN); LI, Xiaoke, Shanghai 200335 (CN)
(74) Representative: Tansley, Sally Elizabeth
(86) International application number: PCT/EP2013/062359
(87) International publication number: WO 2014/009099

(56) References cited:
- US-A1- 2002 041 853
- US-A1- 2011 171 145
- Marek Kosmulski: "Evaluation of points of zero charge of aluminum oxide reported in the literature", , 1 January 2001 (2001-01-01), XP055051072, Retrieved from the Internet: URL:http://www.minproc.pwr.wroc.pl/journal /pdf/2001 sem/str5-14.pdf [retrieved on 2013-01-24]

## Description

### TECHNICAL FIELD OF THE INVENTION

The present invention relates to oral care compositions such as tooth pastes, gums, mouthwashes and the like. In particular the present invention relates to oral care compositions containing particles having a refractive index of at least 1.9 and an isoelectric point of greater than pH 6.5. The invention also relates to the use of such compositions for whitening teeth.

### BACKGROUND TO THE INVENTION

Products we enjoy as consumers, unfortunately, often have a negative impact on our teeth. Acidic drinks and sweets, for example, can result in tooth erosion by attacking enamel that coats and protects the teeth. Moreover, tobacco based products as well as beverages like coffee and tea can stain teeth and thereby result in a smile that is often not attractive.

Products that address whitening have been developed. Conventional products often comprise peroxides, coarse abrasives or both.

International patent application published as US 2011/171145 (Garrett Caldwell) discloses a tooth whitening composition for contacting a tooth in an oral cavity, comprising of hydrogen peroxide, a thickening agent, a flavour enhancer, a sweetening/flavour masking agent, titanium dioxide and mica, and a balance of RO water or distilled water.

These types of products are often not desired since they can sometimes be perceived to cause damage to teeth and gums if improperly used. Moreover, such products can be expensive and are not attractive to lower income consumers in need of tooth whitening.

Compositions have also been developed for use as tooth fillings or which otherwise solidify in or around teeth owing to the presence of cement, resins, photopolymerizing monomers, high molecular weight gelling polymers and the like. Such compositions remain in contact with teeth for extended periods and are said to aid whitening.

European patent application published as EP 1 550 428 A (KURARAY MEDICAL INC) discloses a dental coating kit with high adhesiveness to teeth that contains a primer composition including an acidic group-containing polymeric monomer (a), water (b) and a water-soluble solvent (c) and a surface smoothing composition including a polyfunctional polymeric monomer (f), a volatile solvent (g) and a photopolymerization initiator (h); and a dental coating kit with high adhesiveness to teeth and minimally suffering from chipping and peeling off that contains a primer composition including an acidic group-containing polymeric monomer (a), water (b) and a water-soluble solvent (c), a coating composition including a polymeric monomer (d) and a photopolymerization initiator (e), and a surface smoothing composition including a polyfunctional polymeric monomer (f), a volatile solvent (g) and a photopolymerization initiator (h). Either dental coating kit is said to be useful as a kit for preventing stain and color return of a bleached tooth.

Unfortunately such compositions may still require the use of harsh oxidizing agents, such as peroxides and/or require the entire solid composition to remain in contact with the teeth for extended periods of time. Thus such compositions may not be suitable to use in a consumer's daily routine of cleaning teeth for a few minutes with dentifrice.

The present inventors have therefore recognized that there is a need to develop an oral care product that is suitable to whiten teeth while at the same time being gentle for use and/or affordable for a broad range of consumers and/or effective when used as part of daily teeth- cleaning or mouth-washing routines. This invention, therefore, is directed to an oral care composition as well as a method for whitening teeth.

### TESTS AND DEFINITIONS

### Dentifrice

"Dentifrice" for the purposes of the present invention means a paste, powder, liquid, gum or other preparation for cleaning the teeth or other surfaces in the oral cavity.

### Tooth Paste

"Tooth paste" for the purposes of the present invention means a paste or gel dentifrice for use with a toothbrush. Especially preferred are tooth pastes suitable for cleaning teeth by brushing for about two minutes.

### Mouth Wash

"Mouth wash" for the purposes of the present invention means liquid dentifrice for use in rinsing the mouth. Especially preferred are mouth washes suitable for rinsing the mouth by swishing and/or gargling for about half a minute before expectorating.

### Refractive Index

Refractive index is quoted at a temperature of 25 °C and a wavelength of 589 nm.

### Isoelectric Point

Isoelectric point is defined as the pH at which particles have zero net charge (at 25 °C). For an oral care composition, the presence of particles having an isoelectric point above a specified pH value can be confirmed by deposition onto an anionic surface of particles from slurry of the oral care composition in an aqueous medium buffered at the specified pH and not comprising anions (such as phosphate) which bind to cationic particles. For example, the presence of particles having an isoelectric point above pH 7.0 in an oral care composition can usually be confirmed by contacting a slurry of the oral care composition in Tris-HCI buffer of pH 7.0 with a silica glass slide; rinsing the slide with water; and then comparing the opacity of the slide before and after treatment. Deposition of particles results in a noticeable increase in opacity (decrease in transmittance). For example, the transmittance of light having a wavelength of 700 nm through the slide may be decreased by at least 5%, more preferably at least 10% and most preferably from 15 to 50% after treatment.

### Particle Size

Particle size as used herein refers to particle diameter unless otherwise stated. Diameter is meant to mean the largest measurable distance on a particle in the event a well-defined sphere is not generated. For polydisperse samples, diameter means the z-average particle size measured, for example, using dynamic light scattering (see international standard ISO 13321) with an instrument such as a Zetasizer Nano™ (Malvern Instruments Ltd, UK).

### pH

When referring to the pH of an oral care composition, this means the pH measured when 1 part by weight of the composition is uniformly dispersed and/or dissolved in 20 parts by weight pure water at 25 °C. In particular the pH may be measured by manually mixing 1 g oral care composition with 20 ml water for 30 s, then immediately testing the pH with indicator paper or a pH meter.

### Oxidative Whitening Compound

"Oxidative whitening compound", as used herein, means one or more of peroxides, perborates, percarbonates, peroxyacids, persulfates and metal chlorites.

### Substantially Free

"Substantially free of', as used herein, means less than 1.5%, and preferably less than 1.0%, and more preferably less than 0.75% and more preferably still less than 0.5%, and even more preferably less than 0.1 % and most preferably from 0.0 to 0.01% by weight, based on total weight of the oral care composition, including all ranges subsumed therein.

### Viscosity

Viscosity of a tooth paste is the value taken at room temperature (25 °C) with a Brookfield Viscometer, Spindle No. 4 and at a speed of 5 rpm. Values are quoted in centipoises (cP = mPa.s) unless otherwise specified.

### Miscellaneous

Except in the examples, or where otherwise explicitly indicated, all numbers in this description indicating amounts of material or conditions of reaction, physical properties of materials and/or use may optionally be understood as modified by the word "about".

All amounts are by weight of the final oral care composition, unless otherwise specified.

It should be noted that in specifying any range of values, any particular upper value can be associated with any particular lower value.

For the avoidance of doubt, the word "comprising" is intended to mean "including" but not necessarily "consisting of' or "composed of'. In other words, the listed steps or options need not be exhaustive.

The disclosure of the invention as found herein is to be considered to cover all embodiments as found in the claims as being multiply dependent upon each other irrespective of the fact that claims may be found without multiple dependency or redundancy.

Where a feature is disclosed with respect to a particular aspect of the invention (for example a composition of the invention), such disclosure is also to be considered to apply to any other aspect of the invention (for example a method of the invention) *mutatis mutandis.*

### SUMMARY OF THE INVENTION

In a first aspect, the present invention is directed to an oral care composition according to claim 1 comprising:
a) particles having a refractive index of at least 2.2 and an isoelectric point of greater than pH 6.5; and
b) physiologically acceptable carrier.

Such a composition is found to be beneficial for whitening teeth and so in a further aspect the present invention provides a method of increasing the whiteness of the teeth of an individual comprising applying the composition of the first aspect to at least one surface of the teeth.

In a yet further aspect, the present invention provides use of a composition according to the claims, comprising particles having a refractive index of at least 2.2 and an isoelectric point of greater than pH 6.5 for whitening teeth, preferably for whitening the enamel of teeth.

### DETAILED DESCRIPTION

The composition of the present invention comprises particles having a refractive index of at least 2.2 and an isoelectric point of greater than pH 6.5. Particles having such properties are found to deposit on tooth surfaces (especially enamel) even during routine oral hygiene practices such as brushing, to effectively whiten teeth.

In order to provide more efficient whitening, it is preferable to use particles with even higher refractive index. Thus the particles preferably have a refractive index of at least 2.2, even more preferably at least 2.3, even more preferably still at least 2.4 and most preferably at least 2.5. The maximum refractive index of the particles is not particularly limited but preferably the particles have a refractive index up to 3.0, more preferably up to 2.9.

Suitable materials to provide such a high refractive index are limited only in that they should be suitable for use in oral hygiene applications. Particularly suitable are metal salts and preferably the particles comprise a metal salt. Preferred are salts where the metal is selected from zinc (Zn), titanium (Ti) or a combination thereof. Additionally or alternatively the metal salt is (or at least comprises) a metal oxide. Especially preferred metal salts are zinc oxide (ZnO), titanium dioxide (TiO₂) or a combination thereof, and the particles preferably comprise ZnO, TiO₂ or a combination thereof in an amount of at least 50% by weight of the particles, more preferably at least 70%, more preferably still from 80 to 100% and most preferably from 85 to 95%. Most preferred, owing to its high refractive index and suitability for oral application is titanium dioxide (TiO₂). Of the crystal forms of titanium dioxide, rutile is the most preferred owing to its high refractive index, thus in a most preferred embodiment the TiO₂ comprises or is rutile.

The relatively high isoelectric point of the particles for use in the invention allows that they may carry some positive charge when in contacted with oral fluid (which has a pH of around 7). Without wishing to be bound by theory, the present inventors believe that the positive charge of the particles allows for enhanced interaction with and deposition on tooth surfaces. In order that the positive charge in the oral cavity is even higher, the particles preferably have an isolelectric point greater than pH 7.0, more preferably greater than 7.5, more preferably still greater than 8.0, and most preferably in the range 8.5 to 12.0.

Certain materials may naturally have a high isoelectric point. However in some instances it may be desirable to coat the particles with a material having a high isoelectric point. Thus in some embodiments the particles comprise a core of a first material and a coating of a second material where the isoelectric point of the second material is greater than that of the first material. Typically the first material will have a refractive index higher than the refractive index of the second material. The first material will typically form the majority of the particle and so preferably the particles comprise the first material and the second material in a weight ratio (first material: second material) of at least 1:1, more preferably at least 2:1, more preferably still at least 3:1 and most preferably in the range 4:1 to 50:1.

Examples of materials which naturally have a high isoelectric point and so are useful as the second material of the coated particles, include one or more of alumina (Al₂O₃), copper oxide, lanthanum oxide, nickel oxide, lead oxide, zinc oxide, magnesium oxide. Especially preferred as the second material is alumina, owing to its wide availability and high isoelectric point.

Specific examples of coated particles for use in the present invention include zinc oxide and/or titanium dioxide particles coated with alumina (Al₂O₃). Titanium dioxide particles coated with alumina are available commercially, for example, from suppliers like DuPont under the trade name Ti-Pure®. Ti-Pure® R-960 is often preferred for use as the particles in the present invention.

The particles for use in the present invention are preferably not so large as to be visible as individual specks when applied on teeth. Preferably therefore, the particles have a size of less than 100 µm, more preferably less than 50 µm, more preferably still less than 10 µm and most preferably less than 5 µm. Smaller particles also benefit from larger surface area per unit mass, thus allowing for increased interaction with tooth surfaces. Additionally or alternatively, the particles may have a size of at least 10 nm, more preferably at least 50 nm and most preferably at least 100 nm.

Typically, the oral care composition of the present invention comprises from 0.1 to 60% by weight of the particles, more preferably from 0.2 to 50%. Even more preferably the composition comprises the particles in an amount of at least 0.3% by weight, more preferably still at least 0.5% or even at least 1%. In a most preferred embodiment the composition comprises the particles in an amount of at least 5% by weight, and optimally in the range 10 to 40% by weight.

In one embodiment of the invention it is preferred that the pH of the oral care composition is less than or equal to the isoelectric point of the particles to allow for the particles to be positively charged immediately on introduction to the oral cavity. More preferably the oral care composition has a pH at least 0.5 pH units less than the isoelectric point of the particles, even more preferably from 1 to 4 pH units less than the isoelectric point of the particles. Additionally or alternatively the pH of the oral care composition is preferably less than 7.5, more preferably at less than 7.0 and most preferably in the range 6.5 to 5.0.

Conventional oral care compositions typically comprise several materials which bring benefits to teeth and/or help provide a consumer-acceptable product (e.g. in terms of stability and/or rheology). Some of these materials ("anion sources") may themselves comprise anions which carry a net negative charge when present in the oral care formulation. If the particles of the present invention prematurely react which such anions their isoelectric point may be lowered resulting in impairment of their ability to carry a positive charge in the oral cavity and effectively deposit on teeth.

One solution to this problem is to provide the oral care composition with a relatively high pH such that the particles are not in a positively charged state before use. Thus in some embodiments the oral care composition preferably has a pH greater than the isoelectric point of the particles. More preferably the oral care composition has a pH at least 0.5 pH units greater than the isoelectric point of the particles, even more preferably from 1 to 4 pH units greater than the isoelectric point of the particles. Additionally or alternatively the pH of the oral care composition is preferably at least 7.5, more preferably at least 8.0 and most preferably in the range 8.5 to 10.5.

Additionally or alternatively the amount of anion-source material in the composition may be controlled such that little or no interaction with the particles occurs.

One common anion source is phosphate-containing compounds. Illustrative examples of the types of phosphate-containing compounds used in oral care compositions include monosodium phosphate, sodium dihydrogenphosphate, disodium hydrogenphosphate, sodium pyrophosphate, tetrasodium pyrophosphate, sodium hexametaphosphate, monopotassium phosphate, potassium dihydrogenphosphate, dipotassium hydrogenphosphate, trisodium phosphate, tripotassium phosphate, mixtures thereof or the like. Preferably the composition is substantially free from phosphate-containing compounds.

The composition of the present invention comprises physiologically acceptable carrier. Typically the carrier comprises at least surfactant, thickener, humectant or a combination thereof.

Preferably the composition comprises a surfactant. Preferably the composition comprises at least 0.01 % surfactant by weight of the oral care composition, more preferably at least 0.1% and most preferably from 0.5 to 7%.

Many oral care compositions comprise anionic surfactants and in some embodiments the composition of the invention may comprise anionic surfactant. Examples of anionic surfactants include the sodium, magnesium, ammonium or ethanolamine salts of C₈ to C₁₈ alkyl sulphates (for example sodium lauryl sulphate), C₈ to C₁₈ alkyl sulphosuccinates (for example dioctyl sodium sulphosuccinate), C₈ to C₁₈ alkyl sulphoacetates (such as sodium lauryl sulphoacetate), C₈ to C₁₈ alkyl sarcosinates (such as sodium lauryl sarcosinate), C₈ to C₁₈ alkyl phosphates (which can optionally comprise up to 10 ethylene oxide and/or propylene oxide units) and sulphated monoglycerides. However, as explained above, in certain embodiments it is desirable that the oral care composition is substantially free from anion sources. Therefore the oral care composition is preferably substantially free from anionic surfactant.

The surfactant preferably comprises cationic surfactant, non-ionic surfactant or a mixture thereof. Suitable nonionic surfactants include optionally polyethoxylated fatty acid sorbitan esters, ethoxylated fatty acids, esters of polyethylene glycol (such as alkylphenol polyoxyethylene ether), ethoxylates of fatty acid monoglycerides and diglycerides, and ethylene oxide/propylene oxide block polymers (such as poloxamer). Other suitable surfactants include amphoteric surfactants, such as betaines or sulphobetaines. Mixtures of any of the above described materials may also be used.

Most preferred are non-ionic surfactants, especially poloxamer, alkylphenol polyoxyethylene ether or a mixture thereof.

Thickener may also be used in this invention and is limited only to the extent that the same may be added to a composition suitable for use in the mouth. Illustrative examples of the types of thickeners that may be used in this invention include, sodium carboxymethyl cellulose (SCMC), hydroxyl ethyl cellulose, methyl cellulose, ethyl cellulose, gum tragacanth, gum Arabic, gum karaya, sodium alginate, carrageenan, guar, xanthan gum, Irish moss, starch, modified starch, silica based thickeners including silica aerogels, magnesium aluminum silicate (e.g., Veegum), Carbomers (cross-linked acrylates) and mixtures thereof. However, commonly used thickeners, such as carboxymethyl cellulose and/or carbomer are anionic. In some embodiments it is preferred that the composition is substantially free from anionic polymers.

Thickener typically makes up from 0.01 to about 10%, and preferably, from 0.1 to 8%, and most preferably, from 1.5 to 6% by weight of the oral care composition, based on total weight of the composition and including all ranges subsumed therein.

When the composition of this invention is a toothpaste, the same typically has a viscosity from about 50,000 to 180,000 centipoise, and more preferably, from 60,000 to 170,000 centipoise, and most preferably, from 65,000 to 165,000 centipoise.

Suitable humectants are preferably used in the oral care composition of the present invention and they include, for example, glycerin, sorbitol, propylene glycol, dipropylene glycol, diglycerol, triacetin, mineral oil, polyethylene glycol (preferably, PEG-400), alkane diols like butane diol and hexanediol, ethanol, pentylene glycol, or a mixture thereof. Glycerin, polyethylene glycol, sorbitol or mixtures thereof are the preferred humectants.

The humectant may be present in the range of from 10 to 90% by weight of the oral care composition. Preferably, the humectant makes up from 25 to 80%, and most preferably, from 45 to 70% by weight of the oral care composition, based on total weight of the composition and including all ranges subsumed therein.

Oral care compositions described herein may comprise optional ingredients which are common in the art. These ingredients include antimicrobial agents, anti-inflammatory agents, anti-caries agents, plaque buffers, vitamins, fluoride sources, plant extracts, desensitizing agents, anti-calculus agents, biomolecules, flavors, proteinaceous materials, preservatives, opacifying agents (especially titanium dioxide), coloring agents, pH-adjusting agents, sweetening agents, particulate abrasive materials, polymeric compounds, buffers and salts to buffer the pH and ionic strength of the compositions, and mixtures thereof. Such ingredients typically and collectively make-up less than 20% by weight of the oral care composition described herein, and more preferably, from 0.0 to 15% by weight, and most preferably, from 0.01 to 12% by weight of the composition, including all ranges subsumed therein.

The oral care composition can be used in a method of increasing the whiteness of the teeth of an individual comprising applying the composition to at least one surface of the teeth of the individual. The oral care composition of this invention may additionally or alternatively be for use as a medicament and/or used in the manufacture of a medicament for providing an oral care benefit as described herein, such as for example, for increasing the whiteness of the teeth of an individual.

Typically the composition will be packaged. In tooth paste or gel form, the composition may be packaged in a conventional plastic laminate, metal tube or a single compartment dispenser. The same may be applied to dental surfaces by any physical means, such as a toothbrush, fingertip or by an applicator directly to the target area. In liquid mouthwash form the composition may be packaged in a bottle, sachet or other convenient container.

The composition can be effective even when used in an individual's daily oral hygiene routine. For example, the composition may be brushed onto the teeth and/or be rinsed around the inside of the mouth of the individual. The composition may, for example, be contacted with the teeth for a time period of one second to 20 hours. More preferably from 10 s to 10 hours, more preferably still from 30 s to 1 hour and most preferably from 1 minute to 5 minutes. The composition may be used daily, for example for use by an individual once, twice or three times per day.

The following examples are provided to facilitate an understanding of the present invention. The examples are not provided to limit the scope of the claims.

### EXAMPLES

### Example 1

A Test toothpaste was prepared according to the formulation information given in Table 1.

**TABLE 1**

| **Material** | **Amount (wt%)** |
|---|---|
| Sorbitol (70 wt%) | 45 |
| Deionised water | 13 |
| PEG 1500 | 2 |
| Abrasive Silica | 8 |
| Particles of TiO₂ coated with Alumina* | 20 |
| Thickening silica | 9 |
| Alkylphenol polyoxyethylene(10) ether (OP-10) | 2 |
| Mint Flavour | 1 |

| | |
|---|---|
| * Ti-Pure® R-960 from DuPont: Particle size of 0.5 µm; Rutile content of 89 wt%; and refractive index of 2.7. | |

The whitening performance of this Test toothpaste was compared with the performance of a regular commercial toothpaste and the performance of a commercial whitening toothpaste.

For testing, 24 bovine teeth were separated into three groups (n=8) and secured in oral impression material. Each group was treated with the test toothpaste, the regular toothpaste or the whitening toothpaste. Treatment involved hand-brushing the teeth with diluted toothpaste (toothpaste: water, 3g : 6g) for 2 min and then incubating the brushed teeth in the slurry of diluted toothpaste for 1 min after brushing. During the following rinse off stage, each group was washed with deionised water three times (16.7 ml each time). The teeth colour was measured using a Minolta 2600D instrument (illuminant, D65/ Observer, 2°) pre and post treatment. The change in tooth whiteness between pre and post treatment was calculated as a whitening index (WIO - see W. Luo et al., "Development of a whiteness index for dentistry", Journal of Dentistry, 2009, 37S, e21-e26).

The results are given in Table 2.

**TABLE 2**

| **Treatment** | **Change in WIO*** | **Standard Deviation** |
|---|---|---|
| Test Toothpaste | 8.8^{A} | 6.5 |
| Regular Commercial Toothpaste | 0.4^{B} | 2.6 |
| Whitening Commercial Toothpaste | 1.0^{B} | 3.9 |

| | | |
|---|---|---|
| *Values with different letter are significantly different (P<0.05). | | |

These results illustrate that even after a single brushing, the test toothpaste can produce significant whitening. In addition the whitening efficacy is superior to that of the commercial toothpastes.

### Example 2

The efficacy of two toothpastes for deposition of titanium dioxide particles on enamel was compared. One toothpaste was the Test toothpaste of Example 1. The other toothpaste (comparative toothpaste) was identical to the test toothpaste except that the particles of TiO₂ coated with alumina were replaced with an identical quantity of uncoated TiO₂ (Hombitan AFDC200).

The two toothpastes, were used respectively on two bovine enamel blocks (3 mm × 3 mm). In treatment, the enamel blocks were hand brushed with diluted toothpaste (toothpaste: water, 3g : 6g) for 2 min and incubated in the slurry of diluted toothpaste for 1 min after brushing. During the following rinse off stage, each block was washed with deionised water three times (3 × 16.7ml). Finally, the enamel blocks were dried in air.

The surface of the dried blocks was characterised using Scanning Electron Microscopy (SEM), and element mapping with Energy Dispersive X-ray Spectroscopy (EDX).

From the SEM micrographs it was apparent that a significant amount of material had been deposited on the enamel surface during brushing either with the test toothpaste or the comparative toothpaste. However, in the corresponding element mapping images more Ti was observed on the surface of the enamel treated with the test toothpaste than on the enamel treated with the comparative toothpaste treatment. Thus the particles which were coated with alumina (and therefore had the highest isoelectric point) were more efficient at depositing on enamel than uncoated titanium dioxide particles.

### Example 3

The whitening efficacy of toothpaste containing three types of particle (each with isoelectric point greater than pH 7) was compared. The toothpaste formulations are given in Table 3 (amounts in wt% of toothpaste).

**TABLE 3**

| **TiO₂ toothpaste** | | **ZnO toothpaste** | | **Al₂O₃ toothpaste** | |
|---|---|---|---|---|---|
| Sorbitol (70wt%) | 10 | Sorbitol (70wt%) | 10 | Sorbitol (70wt%) | 10 |
| Deionised water | 47 | Deionised water | 47 | Deionised water | 47 |
| PEG 1500 | 10 | PEG 1500 | 10 | PEG 1500 | 10 |
| Glycerine | 10 | Glycerine | 10 | Glycerine | 10 |
| Particles of TiO₂ coated with Alumina* | 20 | Particles of Zinc Oxide** | 20 | Particles of Alumina*** | 20 |
| Alkylphenol polyoxyethylene(10) ether | 2 | Alkylphenol polyoxyethylene(10) ether | 2 | Alkylphenol polyoxyethylene(10) ether | 2 |
| Mint Flavour | 1 | Mint Flavour | 1 | Mint Flavour | 1 |

| | | | | | |
|---|---|---|---|---|---|
| *Ti-Pure® R-960 from DuPont: Refractive index of 2.7. **Zinc Oxide (AR) from Sinopharm Chemical Reagent Co. Ltd: Refractive index of 2.0. ***a-Al₂O₃ from Shanghai Chemson Chemicals Co. Ltd: Refractive index of 1.8. | | | | | |

For testing, 30 bovine enamel blocks with clean surfaces were separated into three groups (n=10) and secured in oral impression material. Each group was treated with one of the toothpastes listed in Table 3. Treatment involved hand-brushing the teeth with diluted toothpaste (toothpaste : water, 7 g : 14 g) for 2 min and then incubating the brushed teeth in the slurry of diluted toothpaste for 1 min after brushing. During the following rinse off stage, each group was washed with deionised water two times (20 ml each time). The teeth colour was measured using a Minolta 2600D instrument (illuminant, D65/ Observer, 2°) pre and post treatment. The change in tooth whiteness between pre and post treatment was calculated as a whitening index (WIO).

The results are given in Table 4.

**TABLE 4**

| **Treatment** | **Change in WIO*** | **Standard Deviation** |
|---|---|---|
| TiO₂ Toothpaste | 13.4^{A} | 7.0 |
| ZnO Toothpaste | 3.9^{B} | 1.5 |
| Al₂O₃ Toothpaste | 2.0^{C} | 1.8 |

| | | |
|---|---|---|
| *Values with different letter are significantly different (P<0.05). | | |

These results show that even after one brushing significant differences in tooth whitening between the different toothpastes are apparent. The best whitening efficacy is from the toothpaste containing the coated TiO₂ particles.

Some of the brushed enamel blocks were also analysed by SEM and EDX. The SEM micrographs showed that in each case many particles were deposited on the enamel surface. The elemental analysis also confirmed large amounts of deposited titanium (for the TiO₂ toothpaste), zinc (for the ZnO toothpaste) or aluminium (for the Al₂O₃ toothpaste).

### Example 4

The deposition of particles from toothpaste on to the surface of silica glass was investigated. All procedures and tests were performed at room temperature (25 °C).

The toothpaste formulations are given in Table 5 (amounts in wt% of toothpaste).

**TABLE 5**

| **Test toothpaste** | | **Control toothpaste** | |
|---|---|---|---|
| Sorbitol (70wt%) | 45 | Sorbitol (70wt%) | 45 |
| Deionised water | 13 | Deionised water | 13 |
| PEG 1500 | 2 | PEG 1500 | 2 |
| Abrasive Silica | 8 | Abrasive Silica | 8 |
| Particles of TiO₂ coated with Alumina* | 20 | Particles of uncoated TiO₂** | 20 |
| Thickening Silica | 9 | Thickening Silica | 9 |
| Alkylphenol polyoxyethylene(10) ether | 2 | Alkylphenol polyoxyethylene(10) ether | 2 |
| Mint Flavour | 1 | Mint Flavour | 1 |

| | | | |
|---|---|---|---|
| *Ti-Pure® R-960 from DuPont. **AFDC200 from Hombitan. | | | |

Cover glass slides were used as substrate and had a length of 18 mm, width 18 mm, and thickness of 0.15 mm (100PCS, Sail Brand, Made in China). The glass slides were cleaned with the following pre-treatment: White Cat® brand detergent was used to primarily remove oil on cover glass surface under sonication. After rising with deionised water, all the cover glasses were then washed with acetone, followed by a further water rinse. The cover glasses were then soaked in highly concentrated nitric acid for 30 mins (HNO₃ >95% from Sinopharm Chemical Reagent Co., Ltd). Finally, the cover glasses were soaked in deionised water under sonication for at least 15 mins. The pre-treated cover glasses were stored in ethanol until use.

For testing, each cover glass slide was soaked in a slurry of 2.00 g toothpaste and 20 ml 0.05 M Tris-HCI buffer (pH 7.02) for 20 mins, followed by three time deionised water rinse (20 ml each rinse). The glass slides were then dried at 40 °C in an oven. Before and after the toothpaste treatment, the cover glass' transmission spectra were obtained using a UV-Vis Perkin Elmer Lambda 650S spectrometer (PE650S) and wherein the light path was through the thickness of the slide (i.e. path length of 0.15 mm).

In the following step, each treated, dried cover glass was incubated in 10 ml 0.1 M phosphate buffer (pH 7.00) for 2 mins under sonication. The sonicated buffer was collected and used for particle size and zeta potential characterization using a Malvern Nano-ZS.

The Transmittance measurements for the glass slides at various stages in the experiment are shown in Table 6.

**TABLE 6**

| **Toothpaste** | **Treatment** | **Transmittance (%) at wavelength of:** | | | |
|---|---|---|---|---|---|
| | | **400 nm** | **500 nm** | **600 nm** | **700 nm** |
| **Control** | None | 93.4 | 94.4 | 95.0 | 94.8 |
| | Slurry-rinse-dry | 93.2 | 93.7 | 94.3 | 94.4 |
| | Sonication in phosphate buffer | 93.6 | 94.1 | 94.5 | 94.5 |
| **Test** | None | 93.5 | 94.5 | 95.0 | 94.7 |
| | Slurry-rinse-dry | 75.7 | 76.6 | 76.9 | 78.0 |
| | Sonication in phosphate buffer | 93.6 | 94.1 | 94.3 | 94.2 |

The data in Table 6 demonstrate that there is hardly any change in opacity of the cover slides treated with the control toothpaste at any stage. Thus we can conclude that very little of the uncoated TiO₂ deposited on the glass surface.

On the other hand, treatment of the glass slides with the Test toothpaste decreased the glass transmittance by at least 15% at all wavelengths, indicating deposition of the coated TiO₂ onto cover glass. In the following step of sonication, the transmission was restored to its original value presumably because the coated TiO₂ particles bind to the phosphate anions in the buffer in preference to the glass surface. Analysis of the phosphate buffer after sonication showed a particle size distribution with a peak at around 700 nm which is close to the manufacturer's quoted particle size of 0.5 µm. In the zeta potential characterization, the surface charge of the particles detected in the sonicated phosphate buffer was -25.4 mV, indicating that if the particles were indeed the coated TiO₂ particles then they had become coated with anions in the phosphate buffer.

## Claims

1. An oral care composition comprising:
a) particles having a refractive index of at least 2.2 and an isoelectric point of greater than pH 6.5; and
b) physiologically acceptable carrier.
wherein the refractive index is quoted at a temperature of 25°C and a wavelength of 589 nm and isoelectric point is defined at a temperature of 25°C;
wherein the presence of particles having an isoelectric point above a specified pH value can be confirmed by deposition onto an anionic surface of particles from slurry of the oral care composition in an aqueous medium buffered at the specified pH and not comprising anions which bind to cationic particles;
wherein the particles comprises a metal oxide;
wherein the particles have a size in the range of from 50 nm to 10 µm; and wherein the carrier comprises at least surfactant, thickener, humectant or a combination thereof.

2. The oral care composition as claimed in claim 1, wherein the refractive index of the particles is in the range 2.3 to 2.9.

3. The oral care composition as claimed in claim 1 or claim 2, wherein the isoelectric point of the particles is greater than 7.0, preferably in the range 7.5 to 12.0.

4. The oral care composition as claimed in any of the preceding claims, wherein the metal oxide is TiO₂.

5. The oral care composition as claimed in claim 4, wherein the particles comprise TiO₂ in an amount of at least 50% by weight of the particles.

6. The oral care composition as claimed in claim 4 or claim 5, wherein the TiO₂ is coated with at least one material selected from alumina, copper oxide, lanthanum oxide, nickel oxide, lead oxide and magnesium oxide, preferably coated with alumina.

7. The oral care composition as claimed in any one of the preceding claims, wherein the particles have a size in the range of from 100 nm to 5 µm.

8. The oral care composition as claimed in any one of the preceding claims, wherein the composition is substantially free from phosphate-containing compounds, preferably the total amount of phosphate-containing compounds in the composition is less than 1 wt%.

9. The oral care composition as claimed in any one of the preceding claims, wherein the oral care composition has a pH at least 0.5 pH units less than the isoelectric point of the particles, preferably from 1 to 4 pH units less than the isoelectric point of the particles.

10. The oral care composition as claimed in any one of the preceding claims, wherein the composition comprises cationic surfactant, non-ionic surfactant or a mixture thereof.

11. The oral care composition as claimed in claim 10, wherein the composition comprises non-ionic surfactant, preferably a non-ionic surfactant comprising poloxamer, alkylphenol polyoxyethylene ether or a mixture thereof.

12. The composition as claimed in any one of the preceding claims for use in a method of increasing the whitening of the teeth of an individual comprising applying it to at least one surface of the teeth.

13. A composition as claimed in any one of the preceding claims for use in whitening teeth, preferably for whitening the enamel of teeth.

## Patentansprüche

1. Mundpflegezusammensetzung, umfassend:
a) Partikel, die einen Brechungsindex von mindestens 2,2 und einen isoelektrischen Punkt von mehr als pH 6,5 aufweisen, und
b) einen physiologisch annehmbaren Träger,
wobei der Brechungsindex bei einer Temperatur von 25°C und einer Wellenlänge von 589 nm angegeben und der isoelektrische Punkt bei einer Temperatur von 25°C definiert ist,
wobei die Gegenwart von Partikeln, die einen isoelektrischen Punkt oberhalb eines spezifizierten pH-Wertes aufweisen, durch Ablagerung von Partikeln aus einer Aufschlämmung der Mundpflegezusammensetzung in einem wässrigen Medium, das bei dem spezifizierten pH gepuffert ist und keine Anionen umfasst, die an kationische Partikel binden, auf einer anionischen Oberfläche bestätigt werden kann, wobei die Partikel ein Metalloxid umfassen,
die Partikel eine Größe in dem Bereich von 50 nm bis 10 µm aufweisen und
der Träger mindestens Tensid, Verdickungsmittel, Feuchthaltemittel oder eine Kombination davon umfasst.

2. Mundpflegezusammensetzung, wie im Anspruch 1 beansprucht, wobei der Brechungsindex der Partikel in dem Bereich von 2,3 bis 2,9 liegt.

3. Mundpflegezusammensetzung, wie im Anspruch 1 oder 2 beansprucht, wobei der isoelektrische Punkt der Partikel größer als 7,0 ist, vorzugsweise in dem Bereich von 7,5 bis 12,0 liegt.

4. Mundpflegezusammensetzung, wie in einem der vorhergehenden Ansprüche beansprucht, wobei das Metalloxid TiO₂ ist.

5. Mundpflegezusammensetzung, wie im Anspruch 4 beansprucht, wobei die Partikel TiO₂ in einer Menge von mindestens 50 Gewichts-% der Partikel umfassen.

6. Mundpflegezusammensetzung, wie im Anspruch 4 oder Anspruch 5 beansprucht, wobei das TiO₂ mit mindestens einem Material beschichtet ist, das aus Aluminiumoxid, Kupferoxid, Lanthanoxid, Nickeloxid, Bleioxid und Magnesiumoxid ausgewählt ist, vorzugsweise mit Aluminiumoxid beschichtet ist.

7. Mundpflegezusammensetzung, wie in irgendeinem der vorhergehenden Ansprüche beansprucht, wobei die Partikel eine Größe in dem Bereich von 100 nm bis 5 µm aufweisen.

8. Mundpflegezusammensetzung, wie in irgendeinem der vorhergehenden Ansprüche beansprucht, wobei die Zusammensetzung im Wesentlichen frei von Phosphat-haltigen Verbindungen ist, vorzugsweise die Gesamtmenge der Phosphat-haltigen Verbindungen in der Zusammensetzung weniger als 1 Gew.-% beträgt.

9. Mundpflegezusammensetzung, wie in irgendeinem der vorhergehenden Ansprüche beansprucht, wobei die Mundpflegezusammensetzung einen pH von mindestens 0,5 pH-Einheiten weniger als der isoelektrische Punkt der Partikel aufweist, vorzugsweise von 1 bis 4 pH-Einheiten weniger als der isoelektrische Punkt der Partikel.

10. Mundpflegezusammensetzung, wie in irgendeinem der vorhergehenden Ansprüche beansprucht, wobei die Zusammensetzung kationisches Tensid, nicht-ionisches Tensid oder eine Mischung davon umfasst.

11. Mundpflegezusammensetzung, wie im Anspruch 10 beansprucht, wobei die Zusammensetzung nicht-ionisches Tensid umfasst, vorzugsweise ein nicht-ionisches Tensid, das Poloxamer, Alkylphenolpolyoxyethylenether oder eine Mischung davon umfasst.

12. Zusammensetzung, wie in irgendeinem der vorhergehenden Ansprüche beansprucht, zur Verwendung in einem Verfahren zum Steigern des Aufhellens von Zähnen eines Individuums, umfassend deren Auftragen auf mindestens eine Oberfläche der Zähne.

13. Zusammensetzung, wie in irgendeinem der vorhergehenden Ansprüche beansprucht, zum Aufhellen von Zähnen, vorzugsweise zum Aufhellen des Zahnschmelzes von Zähnen.

## Revendications

1. Composition pour le soin oral comprenant :
a) des particules ayant un indice de réfraction d'au moins 2,2 et un point isoélectrique supérieur à pH 6,5 ; et
b) un support physiologiquement acceptable
dans laquelle l'indice de réfraction est coté à une température de 25°C et une longueur d'onde de 589 nm et le point isoélectrique est défini à une température de 25°C ;
dans laquelle la présence de particules ayant un point isoélectrique supérieur à une valeur de pH spécifiée peut être confirmée par dépôt sur une surface anionique de particules à partir d'une suspension de la composition pour le soin oral dans un milieu aqueux tamponné au pH spécifié et ne comprenant pas d'anions qui se lient aux particules cationiques ;
dans laquelle les particules comprennent un oxyde de métal ;
dans laquelle les particules présentent une taille dans l'intervalle de 50 nm à 10 µm ; et
dans laquelle le support comprend au moins un tensioactif, épaississant, humectant ou une combinaison de ceux-ci.

2. Composition pour le soin oral selon la revendication 1, dans laquelle l'indice de réfraction des particules se trouve dans l'intervalle de 2,3 à 2,9.

3. Composition pour le soin oral selon la revendication 1 ou la revendication 2, dans laquelle le point isoélectrique des particules est supérieur à 7,0, de préférence dans l'intervalle de 7,5 à 12,0.

4. Composition pour le soin oral selon l'une quelconque des revendications précédentes, dans laquelle l'oxyde de métal est TiO₂.

5. Composition pour le soin oral selon la revendication 4, dans laquelle les particules comprennent TiO₂ dans une quantité d'au moins 50 % en masse des particules.

6. Composition pour le soin oral selon la revendication 4 ou la revendication 5, dans laquelle le TiO₂ est revêtu avec au moins un matériau choisi parmi l'alumine, l'oxyde de cuivre, l'oxyde de lanthane, l'oxyde de nickel, l'oxyde de plomb et l'oxyde de magnésium, de préférence revêtu avec de l'alumine.

7. Composition pour le soin oral selon l'une quelconque des revendications précédentes, dans laquelle les particules présentent une taille dans l'intervalle de 100 nm à 5 µm.

8. Composition pour le soin oral selon l'une quelconque des revendications précédentes, dans laquelle la composition est pratiquement exempte de composés contenant du phosphate, de préférence la quantité totale de composés contenant du phosphate dans la composition est inférieure à 1 % en masse.

9. Composition pour le soin oral selon l'une quelconque des revendications précédentes, dans laquelle la composition pour le soin oral présente un pH au moins 0,5 unité de pH inférieur au point isoélectrique des particules, de préférence de 1 à 4 unités de pH inférieur au point isoélectrique des particules.

10. Composition pour le soin oral selon l'une quelconque des revendications précédentes, dans laquelle la composition comprend un tensioactif cationique, un tensioactif non-ionique ou un mélange de ceux-ci.

11. Composition pour le soin oral selon la revendication 10, dans laquelle la composition comprend un tensioactif non-ionique, de préférence un tensioactif non-ionique comprenant un poloxamère, un alkylphénol polyoxyéthylène éther ou un mélange de ceux-ci.

12. Composition selon l'une quelconque des revendications précédentes pour une utilisation dans un procédé d'augmentation du blanchiment des dents chez un individu comprenant son application à au moins une surface des dents.

13. Composition selon l'une quelconque des revendications précédentes pour une utilisation dans le blanchiment de dents, de préférence pour le blanchiment de l'émail de dents.
